(19) Europäisches Patentamt European Patent Office Office européen des brevets

(11) **EP 3 445 234 B1**

(12) **EUROPEAN PATENT SPECIFICATION**

(45) Date of publication and mention of the grant of the patent:
**20.07.2022 Bulletin 2022/29**

(21) Application number: **17718357.1**

(22) Date of filing: **18.04.2017**

(51) International Patent Classification (IPC):
**A61B 5/024** (2006.01)      **A61B 5/11** (2006.01)

(52) Cooperative Patent Classification (CPC):
**A61B 5/6844; A61B 5/02416; A61B 5/02438; A61B 5/1118; A61B 5/681; A61B 5/7264;** A61B 5/7221; A61B 2562/0219; A61B 2562/0233; G16H 50/20

(86) International application number:
**PCT/EP2017/059158**

(87) International publication number:
**WO 2017/182456 (26.10.2017 Gazette 2017/43)**

(54) **SYSTEM AND METHOD FOR DETECTING WHEN A SENSOR IS WORN**

SYSTEM UND VERFAHREN ZUR ERFASSUNG, WANN EIN SENSOR GETRAGEN WIRD

SYSTÈME ET PROCÉDÉ PERMETTANT DE DÉTECTER LORSQU'UN CAPTEUR EST PORTÉ

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **18.04.2016 EP 16165813**

(43) Date of publication of application:
**27.02.2019 Bulletin 2019/09**

(73) Proprietor: **Koninklijke Philips N.V.**
**5656 AG Eindhoven (NL)**

(72) Inventors:
• **CREEMERS, Roy**
**5656 AE Eindhoven (NL)**
• **STASSEN, Maurice, Leonardus, Anna**
**5656 AE Eindhoven (NL)**

(74) Representative: **Philips Intellectual Property & Standards**
**High Tech Campus 52**
**5656 AG Eindhoven (NL)**

(56) References cited:
WO-A1-2009/140360      JP-A- 2013 150 847
US-A1- 2002 013 717      US-A1- 2012 215 115
US-A1- 2014 288 390      US-A1- 2015 245 299
US-A1- 2016 004 224      US-A1- 2016 094 899

**Description**

FIELD OF THE INVENTION

This invention relates to a system and method for detecting when a sensor is worn. In particular, it relates to a watch-type sensor which is able to monitor physiological parameters when worn, but which is often removed by a user.

BACKGROUND OF THE INVENTION

Sensors and methods for detecting when a sensor is worn according to the state of the art are for instance described in US2012/0215115A1, US2015/0245299A1, JP2013150847A, US2016/0094899A1, US2014/0288390A1, WO2009/140360A1, US2002/0013717A1 and US2016/0004224A1.
There are known watch-type sensor devices, for example for monitoring heart rate and activity related measurements in respect of the user. These measurements are used to monitor the user's behavior and provide feedback in order to assist the user in achieving goals with respect to a healthier living.
A known personal health monitoring wearable device (watch) of Philips (trade mark) for example comprises a photoplethysmogram (PPG) sensor for heart rate sensing having an LED light source arrangement and a detector for detecting reflected light. The device also has a motion sensor, which for example enables measurement of acceleration, motion cadence and motion speed. The sensed signals may be processed to derive the energy expenditure and also identify an exercise type. The device may also derive the sleep stage of the wearer and the sleeping heart rate, as well as the respiration rate.
To be able to know if measurements are actually from the user of the sensor device, the watch needs to be able to detect if it is being worn or not. If the watch is not worn, any measurements provided by the sensors within the device should be ignored.
It is known for the sensor device to provide elementary wear detection, in particular by detecting if a light path to an optical sensor is blocked or not. In this way, the sensor can detect if the sensor is in a well-lit environment rather than pressed against the skin of the user. This elementary wear detection is however not sufficient to provide accurate wear detection. If, for example, the user takes off the watch and puts it face up on a bedside table, the sensor device will see a material and erroneously think the watch is being worn.
There have been other proposals for implementing detection of when a sensor device is being worn. For example, US 2016/0022175 discloses the use of a motion sensor which is used to detect when the wearable sensor device remains stationary for too long, as an indication of a not-worn state. It also mentions the use of statistical features from other sensors.
There remains a need for a wear detection approach which is easy to implement and also gives reliable results.

SUMMARY OF THE INVENTION

The invention is defined by the claims. In accordance with an aspect of the invention, there is provided a wearable sensor device according to claim 1.
The device interprets both heart rate and activity level information to determine if a sensor is being worn. Activity level information alone may not be sufficient to determine if the sensor is worn. For example a very low activity level may either be because the sensor is not worn and is placed in a stationary location or because the user has very low activity level. Similarly, a very high activity may either be because the user is highly active or because the sensor is placed in a moving location (in a vehicle for example).
Heart rate information alone may also not be sufficient to determine if the sensor is worn. For example a PPG sensor may detect a flow even when not coupled to the user. For example the PPG optical heart rate sensor may pick up signals from ambient lighting or from noise when the watch is not warn. In some known PPG sensor designs, the output of the PPG sensor is set to report a maximum heart rate at its output, in response to such input noise.
The device combines the two measures together with optical sensing to provide a more reliable metric. If there is an unnatural combination of heart rate and activity level, this is an indication that the sensor device is not being worn. By "unnatural" is meant that the combination of physiological parameters would not be expected to arise when the sensor device is in normal use, for example because the two sensor outputs contradict normal physiological function. Thus, an "unnatural" combination of signals is a combination of signals which could not be achieved by a person either when at rest or when exercising. The signals are "unnatural" if they are contrary to the physiological interrelationship between activity level and heart rate, either generally or for the particular user. For example, an activity level is first derived from the motion sensor output. An indication that the sensor device is not worn may then be based on the combination of a heart rate above a threshold and an activity level below a threshold. These two measures are contradictory, in that a higher heart rate should accompany a higher activity level within a given timeframe.

The processor is preferably adapted to process the heart rate monitor output, the motion sensor output and the optical sensor output during a pre-determined time period to determine that the sensor device is not being worn. This pre-determined time period ensures that a reliable detection of the activity level and heart rate may be obtained before making a determination based on the outputs. The pre-determined time period for example is in the range 5 seconds to 1 minute.

The device further comprises an optical sensor for detecting if a skin contact surface of the sensor device is in contact with a surface. This is a known way to detect if the device is being worn. In particular, the sensor device may be determined as not worn when the optical sensor detects that the skin contact surface of the sensor device is not in contact with a surface. The optical sensor functions as an ambient light sensor for sensing ambient light, wherein the ambient light will be occluded when the sensor is being worn. By using this measure in combination with the heart rate and motion sensing, a robust decision making process may be created.

Note that the optical sensor is preferably the optical sensor of an optical heart rate monitor.

The processor categorizes the heart rate monitor, motion sensor and optical sensor outputs to define at least the two states of device worn and device not worn, wherein the transition from the device worn state to the device not worn state is based on analysis of the heart rate monitor, motion sensor and optical sensor outputs and timing values, whereas the transition from the device not worn to the device worn state is based on analysis only of the optical sensor output and timing values.

In this way, once the physiological sensors have been disabled because the device is not worn, only a lower power sensor needs to be used to determine when the physiological sensors need to be reactivated.

The processor is for example adapted to deactivate the collection of sensor data when the sensor device is determined as not being worn. This avoids the collection of spurious data and also saves device power. An alternative is to flag data collected during detection that the device is not worn as unreliable.

The motion sensor may comprise an accelerometer and the heart rate monitor may comprise a PPG sensor.

The device may for example comprise a watch-type sensor device for wearing on the wrist.

Another aspect of the invention is to provide a method of determining whether or not a wearable sensor device is being worn according to claim 8.

The method may comprise providing an indication that the sensor device is not worn based on the combination of a heart rate above a threshold and an activity level below a threshold. These sensed conditions are not expected from a user wearing the device and thus represent spurious signals which may arise when the device is not worn.

The method may further comprise detecting if a skin contact surface of the sensor device is in contact with a surface and if not, providing an indication that the sensor device is not worn. This provides an additional mechanism for detecting if the device is in contact with skin.

The transition between states is selected based on the recognition that the optical sensor gives only true results or false negative results (i.e. the optical sensor may detect a device as worn when it is not, but it will always correctly identify a device as not worn). In this way, the power savings during sensor shut down are increased.

The method may be implemented at least in part in software.

BRIEF DESCRIPTION OF THE DRAWINGS

Examples of the invention will now be described in detail with reference to the accompanying drawings, in which:

Figure 1 shows a watch type sensor device;
Figure 2 shows sensor elements of the sensor device of Figure 1 which are used to determine if the sensor is being worn;
Figure 3 shows a detection method; and
Figure 4 shows a general computer architecture for implementing the detection method.

DETAILED DESCRIPTION OF THE EMBODIMENTS

The invention provides a wearable sensor device according to claim 1. The heart rate monitor output, the motion sensor output and the optical sensor output are processed to determine if the sensor device is currently being worn. In this way, a robust mechanism is provided for detecting when the sensor is being worn. Monitoring for the next transition from a not worn state back to a worn state is achieved with lowest possible power consumption by using only the optical sensing.

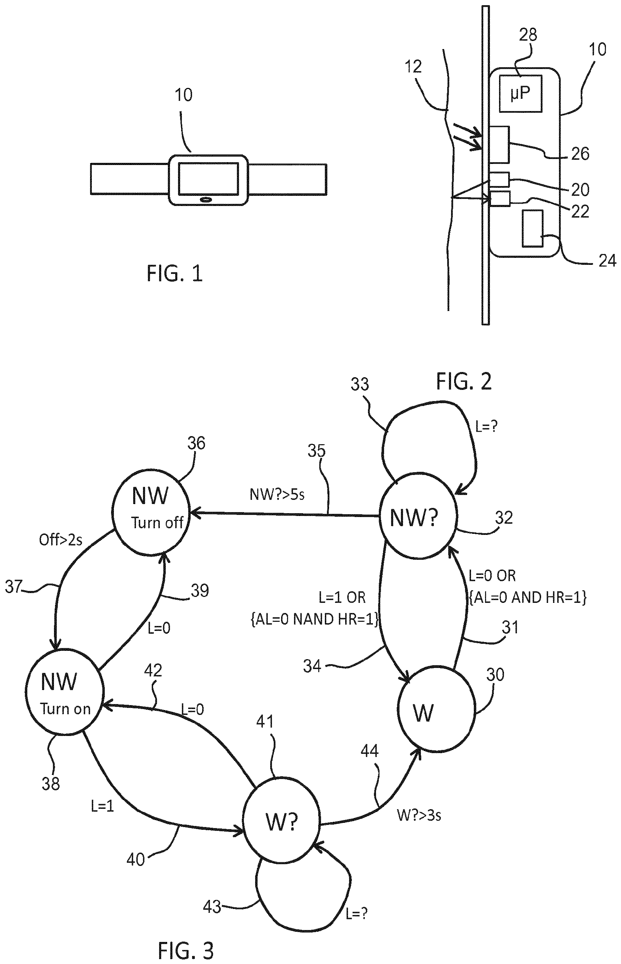

Figure 1 shows a watch type sensor device 10. It is used for monitoring physiological parameters of user, when the sensor device is placed against the wrist.

The sensor device may be used for monitoring various physiological parameters.

A first parameter is the heart rate of the user. This can be measured using a PPG sensor.

A second parameter is an activity level of the user. This can be measured using an accelerometer such as a three-axis accelerometer. Other motion detection sensors may also be used, such as GPS sensors, magnetometers, altimeters and gyroscopes.

The physiological parameters are for example used to provide the user with an indication of their calorie expenditure.

In addition to these physiological parameters, the device has an optical sensor for detecting incident light, and the light sensor is occluded when the device is worn. This may be a dedicated optical sensor or it may be an optical sensor which is already present as part of the PPG sensor. For example, if the light source of the PPG sensor is disabled, then the light detector of the PPG sensor can be used to detect ambient light. This provides a basic way of determining that the sensor device is worn, and there is provided an alternative or additional way of detecting that the sensor is worn.

Figure 2 shows the sensor elements of the sensor device which contribute to the detection of whether or not the device is being worn.

The heart rate monitor comprises a PPG sensor having a light emitter 20 (or plurality of light emitters) and detector 22 for detecting a reflected signal from the skin.

The motion sensor comprises an accelerometer 24 for measuring an activity level. This may for example be measured as the number of acceleration events, each with an acceleration above a threshold, within successive time periods. In this way, activity level is measured as a count of activity events per unit time. For example, there may be a threshold of 45 acceleration events in a 10 second cycle, where the acceleration event is an acceleration spike above a threshold.

For example, an acceleration count may be defined as follows:
An activity count may for example be defined as:

$$EC = \Sigma(x - avg(x)) + \Sigma(y - avg(y)) + \Sigma(z - avg(z))$$

This provides a sum of the deviations of the deviations of the three axis accelerations from the mean. Difference values need to be taken in order to cancel the effect of gravity on the measurements obtained. The summations are carried out during a time window, for example with a sampling rate of 128Hz. In this way, a summation of a fixed number of acceleration values is made during a time window (e.g. 15 seconds), in such a way that the effect of gravity is cancelled.

The light detector 26 provides a signal which is dependent on whether the sensor is occluded by the user. As mentioned above, this alone is not sufficient to provide reliable detection of whether the device is worn, since it does not distinguish between the device being worn and not worn during darkness, or distinguish between the device placed on a table sensor down or being worn.

The device further comprises a controller 28 for processing the sensed signals and also for controlling the sensors, for example placing them into a standby or off mode when sensor data is not needed.

Figure 3 shows one example of the method implemented by the controller 28 in the form of a state diagram.

The states are:

W: the device is detected as being worn;
NW: the device is detected as not being worn;
W?: the device might be worn (i.e. this is an intermediate state between the not worn detected state and the worn detected state);
NW?: the device might not be worn (i.e. this is an intermediate state between the worn detected state and the not worn detected state).

The light detector 26 has two light level thresholds and it provides a decision value L. L=0 is an indication that the device is not worn (i.e. light is received above the higher light threshold). L=1 is an indication that the device is worn (i.e. the light level is below the lower threshold).

In the example shown, there is an intermediate state L=? where a conclusion cannot be reached. This is purely optional and it is a state provided by some known PPG sensor designs.

The heart rate monitor has a heart rate threshold. HR=0 is an indication that the heart rate is below the threshold (or there is no detected heart rate at all) and HR=1 is an indication that the heart rate is above the threshold. The threshold is for example 125 BPM.

The activity level monitor has a threshold activity level. AL=0 is an indication that the activity level is below the threshold (or there is no detected activity at all) and AL=1 is an indication that the activity level is above the threshold.

For the purposes of explanation, the cycle of states will be explained starting with a state in which the device is worn. As will be explained below, this is not however the state of the device on power up.

The first state 30 for the purposes of this explanation is that the device is worn (W).

The algorithm proceeds to a second state 32 (NW?) when there is an indication that the device may have been taken off. This is an intermediate state. The path 31 to state 32 is taken if L=0 (so light is received at the light sensor) or if there is low activity but high heart rate detected. This is the unnatural condition which suggests a spurious data set.

A high heart rate is detected for example if there is a prolonged high heart rate, for example if there is no sample bellow a threshold, such as 125 BPM, within a fixed duration time window, such as a 15s time window.

Similarly, a low activity rate is detected if the activity level metric remains below a threshold throughout a fixed duration time window, again such as a 15s time window.

Low pass filtering may be used to increase the robustness of the data processing.

The algorithm stays in state 32 if the light level state is unknown as shown by path 33 (L=?) or if the light level state remains as L=0.

There is a path 34 back to state 30 if the light level state returns to L=1, or if the contradiction between heart rate and activity level is resolved (i.e. the problem of AL=0 and HR=1 no longer arises). In the latter case, AL=0 NAND HR=1 is true (put another way AL OR NOT(HR) =1).

Note that the algorithm can return to state 30 along path 34 even if the light level gives L=0, as long as the contradiction between heart rate and activity level is resolved. Thus, there are two ways to leave state 30 and two ways to return to it from state 32.

There is a time limit in state 32. If the state remains for more than 5 seconds as shown by path 35 (NW?>5s) the not worn state 36 is confirmed.

In state 36, the sensors are turned off to save power.

Note that this state 36 is the state adopted by the algorithm on powering up.

In one approach, periodically, a new set of sensor data is collected, for the purpose of checking if the not worn state remains valid. As shown by path 37, after 2 seconds (Off>2s) the algorithm moves to state 38. This is another not worn state but the light sensor is turned on.

In another approach, the motion sensor remains turned on even in state 36, and the device only leaves state 36 when any small motion is detected. The light sensor is then turned on.

The light sensor only gives false positives (i.e. it indicates that the device is worn when it is not). It does not give false negatives (i.e. if the device is detected as not worn, it must not be worn because light is reaching the sensor). Thus, all that is needed to leave the not worn state 38 is a check of the light sensor status. If L=0 as shown by path 39 the algorithm returns to state 36 (for another 2 seconds). The physiological sensors do not need to be turned on for this purpose.

If L=1 as shown by path 40 the algorithm proceeds to state 41, which is an intermediate state where it needs to be determined further if the device is be worn..

The algorithm otherwise remains in state 41 while L=1 or is unknown (L=?, path 43). If there is a change back to L=0, the algorithm returns to state 38 along path 42.

If L remains at L=1 or unknown for a set time (3s in this example), the algorithm proceeds along path 44 to the worn state 30.

The timing values and thresholds mentioned above are all simply by way of example.

In general, the example algorithm above provides departure from a detected worn state based on the contradictory heart rate and activity level OR the light detection. The transition from the device worn state 30 to the device not worn states 36,38 is thus based on analysis of the heart rate monitor, motion sensor and optical sensor outputs and also based on timing values.

Once a not worn state has been confirmed, the algorithm provides a departure from the not worn state 38 based only on the light detection. The transition from the device not worn state 38 to the device worn state 30 is based on analysis only of the optical sensor output and also based on timing values.

In this way, the more power consuming physiological sensors are not used again until the worn state is reconfirmed. They are only used to detect when a departure from the worn state is appropriate. In the not worn state these sensors are shut down to save power and prevent recording of erroneous data.

The specific set of states shown is only one example. For example, there may be more than one intermediate state between the detected worn state and the detected not worn state and there may be more than one intermediate state between the between the detected not worn state and the detected worn state.

The algorithm above makes use of light detection as one of three parameters for detecting the worn state. However, the invention may be implemented without the light detector.

For example, a suitable range of values of the heart rate in combination with the contradictory heart rate and activity level information may be used without the need for the light sensing approach.

The system may have further sensors which can contribute to the detection of whether or not the device is being worn. These may include at least one of a skin response sensor, a sound sensor, and a humidity sensor.

In the examples above, it is assumed that a fixed threshold is applied to the heart rate and to the activity level metric, in order to reach the decision of whether the heart rate is low or high, and the activity level is low or high.

These thresholds may in practice be personalized. For example, different people have different rest heart rates, so different thresholds may be appropriate. A threshold is desired which is not reached during normal activity levels. The purpose is to detect false high heart rates, and in some known PPG sensors, these false readings in the presence of noise at the input to the PPG sensor are, by default, the maximum heart rate output of the system. Thus, a high threshold is appropriate in this case, just below the maximum heart rate output level. Furthermore, in such a case, personalization is not needed, since the aim is simply to detect when a maximum heart rate is being reported - but it is inconsistent with the activity level.

In a system which uses only the detection of an "unnatural" combination of signals (and not as an extension to an optical detection system), it may be more useful to provide personalization of the heart rate threshold levels.

The threshold for the activity level may be personalized, but again this is not essential. For example, the purpose of the activity level threshold may simply be to discriminate between a sensor that is absolutely stationary and one that is moving because it is worn. A detected low activity level may simply indicate a stationary sensor. The threshold may thus be just above the noise threshold of the sensor. This is again because the system is only looking for false positives which may arise when the sensor is not worn.

In a system which uses only the detection of an "unnatural" combination of signals (and not as an extension to an optical detection system), it may again be more useful to provide personalization of the activity level threshold levels.

If personalized thresholds are to be used, a running average of previously collected data may be used to set the thresholds, and thereby personalize them to a particular user.

The system described above makes use of a processor for processing the sensed data and for implementing the algorithm described above.

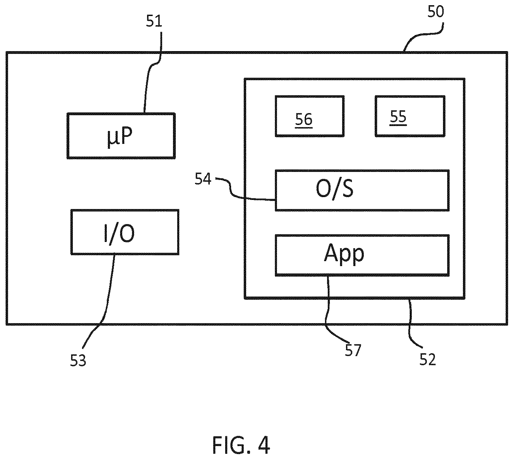

Figure 4 illustrates an example of a computer 50 for implementing the processor described above.

The computer 50 includes, but is not limited to, PCs, workstations, laptops, PDAs, palm devices, servers, storages, and the like. Generally, in terms of hardware architecture, the computer 50 may include one or more processors 51, memory 52, and one or more I/O devices 53 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 51 is a hardware device for executing software that can be stored in the memory 52. The processor 51 can be virtually any custom made or commercially available processor, a central processing unit (CPU), a digital signal processor (DSP), or an auxiliary processor among several processors associated with the computer 50, and the processor 51 may be a semiconductor based microprocessor (in the form of a microchip) or a microprocessor.

The memory 52 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 52 may incorporate electronic, magnetic, optical, and/or other types of storage media. Note that the memory 52 can have a distributed architecture, where various components are situated remote from one another, but can be accessed by the processor 51.

The software in the memory 52 may include one or more separate programs, each of which comprises an ordered listing of executable instructions for implementing logical functions. The software in the memory 52 for example may include one or more of a suitable operating system (O/S) 54, compiler 55, source code 56, and one or more applications 57 in accordance with exemplary embodiments.

The application 57 comprises numerous functional components such as computational units, logic, functional units, processes, operations, virtual entities, and/or modules.

The operating system 54 controls the execution of computer programs, and provides scheduling, input-output control, file and data management, memory management, and communication control and related services.

Application 57 may be a source program, executable program (object code), script, or any other entity comprising a set of instructions to be performed. When a source program, then the program is usually translated via a compiler (such as the compiler 55), assembler, interpreter, or the like, which may or may not be included within the memory 52, so as to operate properly in connection with the operating system 54. Furthermore, the application 57 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 53 may include input devices such as, for example but not limited to, a mouse, keyboard, scanner, microphone, camera, etc. Furthermore, the I/O devices 53 may also include output devices, for example but not limited to a printer, display, etc. Finally, the I/O devices 53 may further include devices that communicate with both inputs and outputs, for instance but not limited to, a network interface controller (NIC) or modulator/demodulator (for accessing remote devices, other files, devices, systems, or a network), a radio frequency (RF) or other transceiver, a telephonic interface, a bridge, a router, etc. The I/O devices 53 also include components for communicating over various networks, such as the Internet or intranet.

When the computer 50 is in operation, the processor 51 is configured to execute software stored within the memory 52, to communicate data to and from the memory 52, and to generally control operations of the computer 50 pursuant to the software. The application 57 and the operating system 54 are read, in whole or in part, by the processor 51, perhaps buffered within the processor 51, and then executed.

When the application 57 is implemented in software it should be noted that the application 57 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The invention is of interest for wearable health monitoring devices generally, and is not limited to devices to be worn on the wrist.

Other variations to the disclosed embodiments can be understood and effected by those skilled in the art, from a study of the drawings, the disclosure, and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference signs in the claims should not be construed as limiting the scope. The invention is defined in the appended claims.

**Claims**

1.  A wearable sensor device, comprising:

    a heart rate monitor (20,22) having an output representing a monitored heart rate;
    a motion sensor (24) having an output representing a sensed motion;
    an optical sensor (26) for detecting if a skin contact surface of the sensor device is in contact with a surface; and
    a processor (28),
    wherein the processor is adapted to process the heart rate monitor output, the motion sensor output and the optical skin contact sensor output and to determine that the sensor device is currently not being worn based on analysis of the combination of output signals from the heart rate monitor, motion sensor and optical skin contact sensor,
    wherein the processor is adapted to categorize the heart rate monitor, motion sensor and optical skin contact sensor outputs to define at least the two states of device worn (30) and device not worn (38),
    wherein the processor is adapted to implement a transition from the device worn state (30) to the device not worn state (38) based on analysis of the heart rate monitor, motion sensor and optical skin contact sensor outputs and timing values, and
    **characterised in that** the processor is adapted to implement a transition from the device not worn state (38) to the device worn state (30) based on analysis only of the optical skin contact sensor output and timing values such that only the optical skin contact sensor output can trigger a transition from the device not worn state (38)..

2.  A device as claimed in claim 1, wherein the processor is adapted to process the heart rate monitor output, the motion sensor output and the optical sensor output during a pre-determined time period to determine that the sensor device is not being worn.

3.  A device as claimed in claim 1, wherein the processor (28) is adapted to derive an activity level from the motion sensor output.

4.  A device as claimed in claim 3, wherein the processor (28) is adapted to provide an indication that the sensor device is not worn based on the combination of a heart rate monitor output above a threshold and an activity level below a threshold.

5.  A device as claimed in any preceding claim, wherein the heart rate monitor is an optical heart rate monitor and the optical sensor is an optical sensor of the optical heart rate monitor.

6.  A device as claimed in any preceding claim, wherein the processor (28) is adapted to deactivate the collection of sensor data when the sensor device is determined as not being worn.

7. A device as claimed in any preceding claim, wherein the motion sensor (24) comprises an accelerometer and the heart rate monitor (22,24) comprises a PPG sensor.

8. A computer-implemented method of determining whether or not a wearable sensor device is being worn, comprising:

monitoring a heart rate using a heart rate monitor (20,22) of the wearable sensor device;
sensing an activity level using a motion sensor (24) of the wearable sensor device;
detecting if a skin contact surface of the sensor device is in contact with a surface using an optical sensor (26) of the wearable sensor device;
processing the heart rate, the activity level and the optical skin contact sensor output to determine that the sensor device is currently not being worn based on a combination of output signals from the heart rate monitor, motion sensor and optical skin contact sensor;
categorizing the heart rate monitor, motion sensor and optical skin contact sensor outputs to define at least the two states of device worn (30) and device not worn (38); and
carrying out the transition from the device worn state (30) to the device not worn state (38) based on analysis of the heart rate monitor, motion sensor and optical skin contact sensor outputs and timing values; **characterised in that** the method further comprises:
carrying out the transition from the device not worn state (38) to the device worn state (30) based on analysis only of the optical skin contact sensor output and timing values such that only the optical skin contact sensor output can trigger a transition from the device not worn state (38).

9. A method as claimed in claim 8, comprising providing an indication that the sensor device is not worn based on the combination of a heart rate monitor output above a threshold and an activity level below a threshold.

10. A method as claimed in claim 8 or 9, further comprising detecting if a skin contact surface of the sensor device is in contact with a surface and if not, providing an indication that the sensor device is not worn.

11. A method as claimed in any one of claims 8 to 10, comprising deactivating the collection of sensor data when the sensor device is determined as not being worn.

12. A computer program comprising computer program code means for implementing the method of any of claims 8 to 11 when said program is run on the processor of the device as claimed in any one of claims 1 to 7.

**Patentansprüche**

1. Tragbare Sensorvorrichtung, die umfasst:

einen Herzfrequenzmesser (20, 22), der einen Ausgang aufweist, der eine überwachte Herzfrequenz darstellt;
einen Bewegungssensor (24), der einen Ausgang aufweist, der eine abgetastete Bewegung darstellt;
einen optischen Sensor (26) zum Erfassen, ob eine Hautkontaktoberfläche der Sensorvorrichtung mit einer Oberfläche in Kontakt ist;
und einen Prozessor (28), wobei der Prozessor dazu angepasst ist, den Ausgang des Herzfrequenzmessers, den Ausgang des Bewegungssensors und den Ausgang des optischen Hautkontaktsensors zu verarbeiten und basierend auf einer Analyse der Kombination von Ausgangssignalen von dem Herzfrequenzmesser, dem Bewegungssensor und dem optischen Hautkontaktsensor zu bestimmen, dass die Sensorvorrichtung aktuell nicht getragen wird, wobei der Prozessor dazu angepasst ist,
die Ausgänge des Herzfrequenzmessers, des Bewegungssensors und des optischen Hautkontaktsensors einzustufen, um mindestens die zwei Zustände, nämlich Vorrichtung wird getragen (30) und Vorrichtung wird nicht getragen (38), zu definieren,
wobei der Prozessor dazu angepasst ist, einen Übergang von dem Zustand Vorrichtung wird getragen (30) zu dem Zustand Vorrichtung wird nicht getragen (38) basierend auf Analyse der Ausgänge und Timingwerten des Herzfrequenzmessers, des Bewegungssensors und des optischen Hautkontaktsensors umzusetzen, und **dadurch gekennzeichnet, dass** der Prozessor dazu angepasst ist,
einen Übergang von dem Zustand Vorrichtung wird nicht getragen (38) zu dem Zustand Vorrichtung wird getragen (30) basierend nur auf Analyse des Ausgangs und der Timingwerte des optischen Hautkontaktsensors derart umzusetzen, dass nur der Ausgang des optischen Hautkontaktsensors einen Übergang von dem Zustand Vorrichtung wird nicht getragen (38) auslösen kann.

2. Vorrichtung nach Anspruch 1, wobei der Prozessor dazu angepasst ist, den Ausgang des Herzfrequenzmessers, den Ausgang des Bewegungssensors und den Ausgang des optischen Sensors während einer vorbestimmten Zeitspanne zu verarbeiten, um zu bestimmen, dass die Sensorvorrichtung nicht getragen wird.

3. Vorrichtung nach Anspruch 1, wobei der Prozessor (28) dazu angepasst ist, ein Aktivitätsniveau aus dem Ausgang des Bewegungssensors abzuleiten.

4. Vorrichtung nach Anspruch 3, wobei der Prozessor (28) dazu angepasst ist, eine Angabe, dass die Sensorvorrichtung nicht getragen wird, basierend auf der Kombination eines Ausgangs des Herzfrequenzmessers oberhalb eines Schwellenwerts und eines Aktivitätsniveaus unterhalb eines Schwellenwerts bereitzustellen.

5. Vorrichtung nach einem vorstehenden Anspruch, wobei der Herzfrequenzmesser ein optischer Herzfrequenzmesser ist und der optische Sensor ein optischer Sensor des optischen Herzfrequenzmessers ist.

6. Vorrichtung nach einem vorstehenden Anspruch, wobei der Prozessor (28) dazu angepasst ist, das Erheben von Sensordaten zu deaktivieren, wenn die Sensorvorrichtung als nicht getragen bestimmt wird.

7. Vorrichtung nach einem vorstehenden Anspruch, wobei der Bewegungssensor (24) einen Beschleunigungsmesser umfasst und der Herzfrequenzmesser (22, 24) einen PPG-Sensor umfasst.

8. Computerimplementiertes Verfahren zum Bestimmen, ob eine tragbare Sensorvorrichtung getragen wird oder nicht, das umfasst:

Überwachen einer Herzfrequenz unter Verwenden eines Herzfrequenzmessers (20, 22) der tragbaren Sensorvorrichtung;
Abtasten eines Aktivitätsniveaus unter Verwenden eines Bewegungssensors (24) der tragbaren Sensorvorrichtung;
Erfassen, ob eine Hautkontaktoberfläche der Sensorvorrichtung mit einer Oberfläche in Kontakt ist, unter Verwenden eines optischen Sensors (26) der tragbaren Sensorvorrichtung;
Verarbeiten der Herzfrequenz, des Aktivitätsniveaus und des Ausgangs des optischen Hautkontaktsensors, um basierend auf einer Kombination von Ausgangssignalen aus dem Herzfrequenzmesser, dem Bewegungssensor und dem optischen Hautkontaktsensor zu bestimmen, dass die Sensorvorrichtung aktuell nicht getragen wird;
Einstufen der Ausgänge des Herzfrequenzmessers, des Bewegungssensors und des optischen Hautkontaktsensors, um mindestens die zwei Zustände Vorrichtung wird getragen (30) und Vorrichtung wird nicht getragen (38) zu definieren;
und Ausführen des Übergangs von dem Zustand Vorrichtung wird getragen (30) zu dem Zustand Vorrichtung wird nicht getragen (38) basierend auf Analyse der Ausgänge des Herzfrequenzmessers, des Bewegungssensors und des optischen Hautkontaktsensors und Timingwerten;
**dadurch gekennzeichnet, dass** das Verfahren weiter umfasst:
Ausführen des Übergangs von dem Zustand Vorrichtung wird getragen (38) zu dem Zustand Vorrichtung wird nicht getragen (30) basierend auf nur Analyse des Ausgangs des optischen Hautkontaktsensors und von Timingwerten derart, dass nur der Ausgang des optischen Hautkontaktsensors einen Übergang von dem Zustand Vorrichtung wird nicht getragen (38) auslösen kann.

9. Verfahren nach Anspruch 8, das das Bereitstellen einer Angabe, dass die Sensorvorrichtung nicht getragen wird, basierend auf der Kombination eines Ausgangs des Herzfrequenzmessers oberhalb eines Schwellenwerts und eines Aktivitätsniveaus unterhalb eines Schwellenwerts umfasst.

10. Verfahren nach Anspruch 8 oder 9, das weiter das Erfassen umfasst, ob eine Hautkontaktoberfläche der Sensorvorrichtung mit einer Oberfläche in Kontakt ist, und falls nicht, Bereitstellen einer Angabe, dass die Sensorvorrichtung nicht getragen wird.

11. Verfahren nach einem der Ansprüche 8 bis 10, das das Deaktivieren des Erhebens von Sensordaten umfasst, wenn die Sensorvorrichtung als nicht getragen bestimmt wird.

**12.** Computerprogramm, das Computerprogrammcodemittel zum Umsetzen des Verfahrens eines der Ansprüche 8 bis 11 umfasst, wenn das Programm auf dem Prozessor der Vorrichtung wie in einem der Ansprüche 1 bis 7 beansprucht ausgeführt wird.

**Revendications**

**1.** Un dispositif de capteur portable, comprend un moniteur de fréquence cardiaque (20,22) qui possède une sortie représentant une fréquence cardiaque surveillée;

un capteur de mouvement (24) possédant une sortie représentant un mouvement détecté;
un capteur optique (26) pour détecter si la surface de contact cutanée du dispositif de capteur est en contact avec une surface; et
un processeur (28),
où le processeur est adapté pour traiter la sortie du moniteur de fréquence cardiaque, la sortie du capteur de mouvement et la sortie du capteur optique de contact cutané et pour déterminer que le dispositif de capteur n'est pas actuellement porté, fondée sur l'analyse de la combinaison des signaux de sortie à partir d'un moniteur de fréquence cardiaque, un capteur de mouvement et le capteur optique de contact avec la peau, où le processeur est adapté pour catégoriser le moniteur de fréquence cardiaque, le capteur de mouvement et les sorties de capteurs optiques de contact cutané pour définir au moins deux états du dispositif porté (30) et du dispositif non porté (38), où le processeur est adapté pour appliquer une transition à partir de l'état d'usure du dispositif (30) à l'état de dispositif non porté (38) fondé sur l'analyse du moniteur de fréquence cardiaque, le capteur de mouvement et les sorties de capteurs optiques à contact cutané et les valeurs de temporisation, et
**caractérisé par le fait que**
le processeur soit adapté pour appliquer une transition à partir d'un dispositif
non porté (38) à l'état de dispositif porté (30) fondé uniquement sur une analyse de la sortie de capteurs optiques à contact cutané et les valeurs de temporisation de sorte que seule la sortie de capteurs optiques à contact cutané peut déclencher une transition à partir du dispositif de l'état de dispositif non porté (38).

**2.** Un dispositif comme revendiqué dans la revendication 1, où le processeur est adapté pour traiter la sortie du moniteur de fréquence cardiaque, la sortie du capteur de mouvement, la sortie de capteurs optiques lors d'une période prédéterminée pour déterminer si le dispositif de capteur n'est pas porté.

**3.** Un dispositif comme revendiqué dans la revendication 1, où le processeur (28) est adapté pour dériver un niveau d'activité à partir de la sortie du capteur du mouvement.

**4.** Un dispositif comme revendiqué dans la revendication 3, où le processeur (28) est adapté pour fournir une indication que le dispositif de capteur n'est pas porté fondé sur la combinaison de la sortie du moniteur de fréquence cardiaque au-dessus d'un seuil et un niveau d'activité en dessous d'un seuil.

**5.** Un dispositif comme revendiqué selon l'une quelconque des revendications précédentes, où le moniteur de fréquence cardiaque est un moniteur optique de fréquence cardiaque et le capteur optique est un capteur optique du moniteur optique de fréquence cardiaque.

**6.** Un dispositif comme revendiqué selon l'une quelconque des revendications précédentes, où le processeur (28) est adapté pour désactiver la collecte des données du dispositif du capteur lorsqu'il est déterminé qu'il n'est pas porté.

**7.** Un dispositif comme revendiqué selon l'une quelconque des revendications précédentes, où le capteur de mouvement (24) comprend un accéléromètre et un moniteur de fréquence cardiaque (22,24) qui comprend un capteur de photopléthysmographe PPG.

**8.** Une méthode exécutée par un ordinateur pour déterminer si un dispositif de capteur portable est porté ou non, comprend:

la surveillance de la fréquence cardiaque en utilisant un moniteur de fréquence cardiaque (20,22) du dispositif de capteur portable;
la détection d'un niveau d'activité en utilisant le capteur de mouvement (24) du dispositif de capteur portable;
détecter si la surface de contact cutanée du dispositif de capteur est en contact avec une surface utilisant un

EP 3 445 234 B1

capteur optique (26) du dispositif de capteur portable;

traiter la fréquence cardiaque, le niveau d'activité et la sortie de capteurs optiques à contact cutané pour déterminer que le dispositif de capteur n'est pas actuellement porté fondé sur une combinaison de signaux de sortie à partir du moniteur de fréquence cardiaque, le capteur de mouvement et le capteur optique à contact cutané;

catégoriser le moniteur de la fréquence cardiaque, le capteur de mouvement et la sortie de capteurs optiques à contact cutané pour

définir au moins deux états du dispositif porté (30) et du dispositif non porté (38); et

exécuter la transition à partir du l'état de dispositif porté (30) à l'état de dispositif non porté (38) fondé sur l'analyse du moniteur de fréquence cardiaque, le capteur de mouvement et les sorties de capteurs optiques à contact cutané et les valeurs de temporisation; **caractérisé par le fait que** la méthode comprend également:

l'exécution de la transition à partir de l'état de dispositif non porté (38) à l'état de dispositif porté (30) fondé uniquement sur l'analyse des sorties de capteurs optiques à contact cutané et les valeurs de temporisation de sorte que la sortie de capteurs optiques à contact cutané peut déclencher une transition à partir de l'état de dispositif non porté (38).

9. Une méthode comme revendiquée dans la revendication 8, comprend la fourniture d'une indication que le dispositif de capteur n'est pas porté, fondé sur la combinaison de la sortie du moniteur de fréquence cardiaque au-dessus d'un seuil et un niveau d'activité en dessous d'un seuil.

10. Une méthode selon la revendication 8 ou 9, comprend également de détecter si la surface de contact cutanée du dispositif de capteur est en contact avec une surface et si ce n'est pas le cas, fournir une indication que le dispositif de capteur n'est pas porté.

11. Une méthode comme revendiquée selon l'une des revendications 8 à 10, comprend la désactivation de la collecte des données du dispositif du capteur lorsque le dispositif de capteur est déterminé comme étant non porté.

12. Un programme informatique comprenant des moyens de codage informatique pour appliquer la méthode selon l'une quelconque des revendications 8 à 11, où ledit programme est exécuté par le processeur du dispositif comme revendiqué selon l'une quelconque des revendications 1 à 7.

FIG. 1

FIG. 2

FIG. 3

FIG. 4

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20120215115 A1
- US 20150245299 A1
- JP 2013150847 A
- US 20160094899 A1
- US 20140288390 A1
- WO 2009140360 A1
- US 20020013717 A1
- US 20160004224 A1
- US 20160022175 A